# EUROPÄISCHE PATENTSCHRIFT

(11) **EP 1 392 173 B1**
(45) Veröffentlichungstag und Bekanntmachung des Hinweises auf die Patenterteilung: **08.02.2006**
(21) Anmeldenummer: 02753061.7
(22) Anmeldetag: 28.05.2002
(51) Int. Cl.: A61B 10/00

(54) **VORRICHTUNG ZUM SAMMELN VON FLÜSSIGEN PROBEN**
DEVICE FOR COLLECTING LIQUID SAMPLES
DISPOSITIF PERMETTANT DE COLLECTER DES ECHANTILLONS LIQUIDES

(30) Priorität: 31.05.2001 DE 10126583
(43) Veröffentlichungstag der Anmeldung: 03.03.2004
(73) Patentinhaber: Envitec-Wismar GmbH, 23966 Wismar (DE)
(72) Erfinder: ECKERMANN, Martin, 18055 Rostock (DE); ULLMANN, Martin, 22399 Hamburg (DE); LINDNER, Bernd, 23626 Ratekau (DE)
(74) Vertreter: Teipel, Stephan
(86) Internationale Anmeldenummer: PCT/EP2002/005868
(87) Internationale Veröffentlichungsnummer: WO 2002/097389

(56) Entgegenhaltungen:
- EP-A- 0 418 739
- WO-A-00/15020
- WO-A-00/76664
- US-A- 2 905 169
- US-A- 2 987 174
- US-A- 5 441 698
- US-A- 5 922 614

## Beschreibung

Die vorliegende Erfindung betrifft eine Vorrichtung zum Sammeln von flüssigen Proben, insbesondere von Körperflüssigkeiten wie Speichel.

Körperflüssigkeiten eigenen sich zum Nachweis von diagnostisch interessanten Substanzen. Diese Substanzen können beispielsweise in Blut in relativ hohen Konzentrationen vorliegen, so daß sie dort leicht nachgewiesen werden können. Andere Substanzen reichern sich im Urin an, der zudem den Vorteil hat, daß er für die Analysezwecke leichter gesammelt werden kann. Darüber hinaus können viele Substanzen wie beispielsweise Antikörper, Antigene, pharmazeutische Wirkstoffe und natürlich auftretende Hormone, aber auch Drogen wie beispielsweise Kokain im Speichel nachgewiesen werden. Für den Nachweis entsprechender Substanzen wurden eine Reihe von Schnelltests entwickelt, die vielfach auf einem einfachen Teststreifen basieren, durch den sich die Flüssigkeitsprobe bewegt und bei Anwesenheit einer nachzuweisenden Substanz beispielsweise eine Farbreaktion auslöst.

Es sind verschiedene Vorrichtungen zum Sammeln entsprechender Flüssigkeitsproben bekannt. Im einfachsten Fall kann beispielsweise ein Teststreifen direkt mit der flüssigen Probe in Kontakt gebracht werden oder der Teststreifen kann wie in der DE 198 30 405 A1 beschrieben mit einer biegestreifen Teststreifenhalterung versehen sein, um den Teststreifen gegenüber mechanischen Beanspruchungen zu schützen. Entsprechende Vorrichtungen eigenen sich jedoch zumeist nicht zum Sammeln beispielsweise von Speichel, da sie zur Vermeidung eines direkten Kontakts mit den Analysereagentien in dem Teststreifen nicht unmittelbar in den Mund genommen werden können. Außerdem wird eine nicht definierte Probenmenge von dem Teststreifen aufgesaugt, so daß es durch Schwankungen der aufgesaugten Probenmenge zu unterschiedlichen Messergebnissen kommen kann.

Eine Vorrichtung zum Sammeln von Speichel mittels eines saugfähigen Kissens ist in der EP-A-0 418 739 offenbart. Diese Vorrichtung umfaßt ein saugfähiges Kissen, das mittels eines Halters in den Mund eingebracht wird. Nachdem das saugfähige Kissen den Speichel aufgenommen hat, muß es bis zur weiteren Untersuchung in einem separaten Behälter aufbewahrt werden. Dazu kann das saugfähige Kissen mittels einer speziellen Vorrichtung von dem Halter getrennt werden. Die beschriebene Vorrichtung zum Sammeln von Speichel besteht aus mehreren Einzelteilen, die separat gehandhabt werden müssen. Außerdem besteht die Möglichkeit, daß der Probennehmer in unerwünschter Weise mit der Speichelprobe der Testperson in Kontakt kommt oder diese unbeabsichtigt verunreinigt. Schließlich ist es für den Probennehmer nur schwierig zu erkennen, ob das saugfähige Kissen eine ausreichende Menge Speichel aufgenommen hat. Dies kann insbesondere dann von Bedeutung sein, wenn die Testperson beispielsweise aufgrund von Drogenkonsum einen verminderten Speichelfluß hat oder eine freiwillige Zusammenarbeit mit dem Probennehmer verweigert.

Um sicherzustellen, daß eine ausreichende Menge Speichel in das saugfähige Kissen einer Speichelsammelvorrichtung aufgenommen wurde, schlägt das US 5,714,341 vor, das saugfähige Kissen mit einem weiteren saugfähigen Teil in dem Griff der Vorrichtung zu versehen, wobei dieser weitere saugfähige Teil eine Indikatorsubstanz enthält. Wenn das saugfähige Kissen mit Speichel getränkt ist, breitet sich der Speichel auch in den zusätzlichen saugfähigen Teil der Vorrichtung aus und transportiert dort einen Farbstoff zu einem Sichtfenster, wodurch eine ausreichende Sättigung des saugfähigen Kissens mit Speichel angezeigt wird. Dies hat jedoch den Nachteil, daß die Testperson potentiell mit dem Farbstoff in Kontakt kommen kann. Außerdem benötigt die offenbarte Speichelsammelvorrichtung eine getrennte Vorrichtung zum Nachweis des gewünschten Analyten, so daß der Probennehmer mit dem Speichel der Testperson in Kontakt kommen kann und insgesamt der Test aufwändig zu handhaben ist.

Eine Vorrichtung zum Sammeln von Speichel, bei der ein saugfähiges Kissen an dem Kolben einer Spritze befestigt ist und das speichelgetränkte Kissen nach Entnahme aus dem Mund mittels des Kolbens in einem Spritzenkörper zur Gewinnung des Speichels ausgepreßt wird, ist in dem US 5,479,937 offenbart. Das US 4,774,962 sieht vor, den in einem saugfähigen Kissen durch Indenmundnehmen des Kissens gesammelten Speichel einer Testperson durch Zentrifugation zu gewinnen. Gemäß dem US 4,418,702 soll der Speichel aus einem entsprechenden speichelgetränkten Kissen mittels einer speziellen Schraubvorrichtung ausgepreßt werden.

Die WO 00/76664 offenbart einen Probenhalter aus einem länglichen Streifen, der an zwei Linien zusammengefaltet werden kann. Der mittlere Abschnitt dieses Streifens weist Felder zur Lagerung biologischer Proben auf.

Die vorstehend beschriebenen Speichelsammelvorrichtungen weisen den Nachteil auf, daß sie aus mehreren Einzelteilen bestehen. Die Handhabung wird daher insbesondere für Routinetests erschwert. Außerdem können durch die Handhabung mehrerer Einzelteile die Probennehmer leicht mit dem speichelgetränkten Kissen in Kontakt kommen. Dies ist zum einen aus Sicht des Probennehmers unerwünscht und erschwert die Akzeptanz entsprechender Routinetests beispielsweise durch die Polizei. Darüber hinaus kann es durch die aufwändige Handhabung zu einer unerwünschten Verunreinigung der Probe kommen. Schließlich ist es schwierig, mit den bekannten Vorrichtungen leicht eine reproduzierbare Speichelmenge zu gewinnen und einem nachfolgenden Test zur Verfügung zu stellen.

Eine Aufgabe der vorliegenden Erfindung besteht somit darin, eine Vorrichtung zum Sammeln von flüssigen Proben und insbesondere von Körperflüssigkeiten wie Speichel zur Verfügung zu stellen, die eine sichere, einfache und hygienische Handhabung auch durch ungeübte Personen ermöglicht. Insbesondere soll ein Kontakt des Probennehmers mit der aufgenommenen flüssigen Probe möglichst vermieden werden. Die Vorrichtung soll ferner einen schnellen Test auf einen gewünschten Analyten ermöglichen ohne daß mehrere Einzelteile getrennt voneinander gehandhabt werden müssen. Die Probe soll in möglichst reproduzierbarer Menge gesammelt und dem Test zur Verfügung gestellt werden. Schließlich soll eine leichte Kontrolle, ob genug Probe von der Vorrichtung aufgenommen wurde, auch für den ungeübten Probennehmer erleichtert werden.

Es wurde nun gefunden, daß eine Vorrichtung zum Sammeln von flüssigen Proben gemäß den Merkmalen des Anspruchs 1 diese Aufgabe löst. Die vorliegende Erfindung betrifft somit eine Vorrichtung zum Sammeln von flüssigen Proben, wobei die Vorrichtung ein saugfähiges Kissen und zwei Gehäuseteile umfaßt, die mit dem saugfähigen Kissen beweglich verbunden sind, wobei die Gehäuseteile derart angeordnet sind, daß in einer ersten Position der Gehäuseteile das saugfähige Kissen die flüssige Probe aufnehmen kann und in einer zweiten Position die Gehäuseteile das saugfähige Kissen umschließen, dadurch gekennzeichnet, daß das saugfähige Kissen an einem Halter befestigt ist, der über mindestens ein Gelenk mit den beiden Gehäuseteilen verbunden ist.

Die erfindungsgemäße Vorrichtung eignet sich insbesondere zum Sammeln von Körperflüssigkeiten und vorzugsweise zum Sammeln von Speichel.

Die erfindungsgemäße Vorrichtung umfaßt ein saugfähiges Kissen und zwei Gehäuseteile, die mit dem saugfähigen Kissen beweglich verbunden sind. Diese bewegliche Verbindung ist derart ausgestaltet, daß die beiden Gehäuseteile in einer ersten Position das saugfähige Kissen freigeben, d.h. nicht umschließen, so daß es an zumindest einer Stelle, vorzugsweise jedoch vollständig von außen zugänglich ist und die flüssige Probe aufnehmen, d.h. aufsaugen kann. In dieser ersten Position können die beiden Gehäuseteile beispielsweise die Funktion eines Griffs ausüben, an dem die Vorrichtung gehalten werden kann, während das saugfähige Kissen in den Mund genommen wird, damit es sich mit Speichel voll saugt.

Die bewegliche Verbindung zwischen dem saugfähigen Kissen und den beiden Gehäuseteilen ist darüber hinaus so ausgestaltet, daß die beiden Gehäuseteile von der vorstehend beschriebenen ersten Position in eine zweite Position bewegt werden können, in der sie das saugfähige Kissen umschließen und so als Gehäuse für das saugfähige Kissen dienen. Das saugfähige Kissen sollte dabei möglichst vollständig von den Gehäuseteilen eingeschlossen werden, vorzugsweise derart, daß die flüssige Probe, die sich in dem saugfähigen Kissen befindet, nicht wieder aus dem so gebildeten Gehäuse austreten kann.

Das saugfähige Kissen ist an einem Halter befestigt, an den die Gehäuseteile angelenkt sind. Hierdurch wird in der ersten Position der Gehäuseteile, d.h. im aufgeklappten Zustand der Vorrichtung, ein Abstand zwischen den beispielsweise als Griff fungierenden Gehäuseteilen und dem saugfähigen Kissen definiert, so daß das saugfähige Kissen leichter in den Mund genommen werden kann.

In der erfindungsgemäßen Vorrichtung sind die beiden Gehäuseteile über mindestens ein Gelenk mit dem Halter verbunden. Dieses Gelenk ist vorzugsweise so ausgestaltet, daß die beiden Gehäuseteile an zumindest einer Stelle klappbar miteinander und mit dem Halter verbunden sind, wobei sich das saugfähige Kissen zwischen den beiden Gehäuseteilen befindet. Dies ermöglicht ein Aufklappen der beiden Gehäuseteile in ihre erste Position, wobei das saugfähige Kissen freigelegt wird. Beim Zuklappen der beiden Gehäuseteile werden diese in ihre zweite Position bewegt, wobei sie das saugfähige Kissen umschließen.

Als Gelenk zwischen den Gehäuseteilen, das eine Bewegung der Gehäuseteile zwischen der ersten und der zweiten Position erlaubt, eignet sich im Prinzip jedes Gelenk, das eine gewünschte Bewegung der Gehäuseteile ermöglicht, wie beispielsweise ein Scharnier mit einem Zapfen, der die beiden Gehäuseteile und den Halter miteinander verbindet. Alternativ kann beispielsweise eine Knickstelle zwischen den beiden Gehäuseteilen z.B. in Form einer Verjüngung der Wandstärke vorgesehen sein. Eine solche Verjüngung läßt sich beispielsweise beim Spritzgießen der Vorrichtung erreichen, wodurch zumindest die beiden Gehäuseteile einstückig in einem Arbeitsschritt erhalten werden können.

Das saugfähige Kissen kann aus jedem Material hergestellt werden, das sich zum Aufsaugen der gewünschten flüssigen Probe eignet. Es kann sich beispielsweise um einen Schwamm, gepreßte Baumwolle oder Cellulose oder ein saugfähiges dickes Papierstück handeln. Vorzugsweise wird das saugfähige Kissen aus gepreßter Cellulose hergestellt. Dies hat den Vorteil, daß ein Streifen aus gepreßter Cellulose in trockenem Zustand flach ist und in nassem Zustand um das zwei- bis dreifache gegenüber dem trockenen Zustand aufquillt. Dies ermöglicht auch dem nichttrainierten Anwender zu erkennen, ob das saugfähige Kissen ausreichend mit der flüssigen Probe vollgesogen ist, insbesondere dann, wenn das saugfähige Kissen beispielsweise im trockenen Zustand etwa so dick wie der Halter ist. Dann läßt sich das Aufquellen des Kissens besonders leicht beobachten. Außerdem nimmt gepreßte Cellulose eine gut definierte Menge an Flüssigkeit auf, so daß über die Größe des saugfähigen Kissens die Probenmenge, die mit der erfindungsgemäßen Vorrichtung aufgenommen wird, relativ genau bestimmbar ist.

Alternativ kann das saugfähige Kissen aus einem anderen Material bestehen, das zum Probesammeln oder -aufnehmen geeignet ist, wie beispielsweise ein Fließmaterial z.B. aus Polyolefin. Ein solches Material weist jedoch gegebenenfalls nicht die Eigenschaft der Volumenzunahme und damit der Probeaufnahmeindikation auf. Als Indikation für eine ausreichende Probeaufnahme können jedoch auch beispielsweise Oberflächeneffekte in dem Halter des saugfähigen Kissens dienen. Vorteilhaft kann hierfür das saugfähige Kissen bis unter eine durchsichtige und aufgeraute Oberfläche des Halters geführt werden. Eine entsprechende Oberfläche ist im trockenen Zustand opak und wird bei Flüssigkeitskontakt klar, so daß ein Klarwerden des entsprechenden Abschnitts des Halters eine ausreichende Probeaufnahme durch das saugfähige Kissen anzeigt. Alternativ kann auch eine Farbstoffindikation wie beispielsweise in dem US 5,714,341 beschrieben als Anzeige für eine ausreichende Probeaufnahme verwendet werden.

Ein geeignetes Material für das saugfähige Kissen kann vom Fachmann leicht gewählt werden. Gepreßte Cellulose wird insbesondere dann bevorzugt, wenn die erfindungsgemäße Vorrichtung zum Sammeln von Speichel aus der Mundhöhle verwendet werden soll. Andere Materialien können dann notwendig sein, wenn der nachzuweisende Analyt inkompatibel zu der gepreßten Cellulose ist, beispielsweise aufgrund einer unspezifischen Absorption etc.

Vorzugsweise liegt die Menge der mit der erfindungsgemäßen Vorrichtung gesammelten flüssigen Probe zwischen 25 und 500 µl, insbesondere zwischen 200 und 400 µl und beispielsweise bei ca. 300 µl. Ebenfalls vorteilhaft ist ein Bereich von 80 bis 250 µl wie z.B. 125 µl. Um reproduzierbare Testergebnisse sicherzustellen, sollte die Fehlertoleranz bei ca. ± 10-20 µl liegen. Dies kann beispielsweise mit einem saugfähigen Kissen aus gepreßter Cellulose erreicht werden, das in trockenem Zustand etwa 1 cm lang, 6 mm breit und 1,5-2 mm dick ist. Die Größe des saugfähigen Kissens ist jedoch letztendlich von der Porengröße und der Materialart abhängig und kann entsprechend vom Fachmann für den gewünschten Zweck ausgewählt werden.

Das saugfähige Kissen kann darüber hinaus ein den Speichelfluß anregendes Mittel umfassen. Als den Speichelfluß anregende Mittel eignen sich beispielsweise Zitronensäure oder Aromen. Eine Ausgestaltung der erfindungsgemäßen Vorrichtung mit einem den Speichelfluß anregenden Mittel ist insbesondere dann von Vorteil, wenn eine Speichelprobe bei Testpersonen mit reduziertem Speichelfluß entnommen werden soll. Ein reduzierter Speichelfluß kann beispielsweise bei Testpersonen auftreten, die bestimmte Drogen zu sich genommen haben. Daher ist die Ausgestaltung der erfindungsgemäßen Vorrichtung mit einem den Speichelfluß anregenden Mittel besonders dann vorteilhaft, wenn die Vorrichtung zum Nachweis von Drogen im Speichel von Testpersonen eingesetzt werden soll.

Das saugfähige Kissen ist über einen Halter mit den beiden Gehäuseteilen verbunden. Die Verbindung zwischen dem saugfähigen Kissen und dem Halter kann im Prinzip beliebig erfolgen. Beispielsweise kann das saugfähige Kissen an dem Halter angeklebt sein oder der Halter kann beim Spritzgießen unmittelbar in einem Schritt an das saugfähige Kissen angegossen werden. In einer weiteren Ausführungsform kann das saugfähige Kissen in eine dafür vorgesehene Aussparung des Halters eingesteckt werden, wobei diese Aussparung dann vorzugsweise eine Einrichtung

wie nach innen gerichtete Zähne aufweist, die ein Herausziehen des saugfähigen Kissens aus der Aussparung verhindert.

In einer besonders vorteilhaften Ausgestaltung umfaßt die erfindungsgemäße Vorrichtung nicht nur die vorstehend beschriebenen Einzelteile zum Sammeln einer flüssigen Probe sondern darüber hinaus auch eine Einrichtung zum Nachweis mindestens eines in der flüssigen Probe nachzuweisenden Analyten. Je nach Art der gesammelten Probe kann es sich um sehr unterschiedliche nachzuweisende Analyten handeln. Handelt es sich bei der Probe um eine Körperflüssigkeit, so kann es beispielsweise wünschenswert sein, in der Probe bestimmte Hormone, Antikörper, pharmazeutische Wirkstoffe und/oder Drogen als Analyten nachzuweisen.

Vorteilhaft handelt es sich bei der Einrichtung zum Nachweis mindestens eines Analyten in der flüssigen Probe um einen oder mehrere Teststreifen (z.B. immunochromatographischer Teststreifen/lateral flow immoassay), wobei jeder Teststreifen für den Nachweis eines oder mehrerer Analyten geeignet sein kann. Entsprechende Teststreifen für verschiedenste Analyten sind dem Fachmann bekannt und können mit der erfindungsgemäßen Vorrichtung kombiniert werden. Beispielsweise kann die erfindungsgemäße Vorrichtung 1, 2, 3, 4 oder 5 Teststreifen enthalten, wobei die Anzahl der Teststreifen im wesentlichen nur von deren Größe, der Größe der erfindungsgemäßen Vorrichtung und der Menge der für jeden Teststreifen benötigten und in dem saugfähigen Kissen zur Verfügung stehenden Probe abhängt. In der Regel wird daher das Probenvolumen bzw. die Größe der Vorrichtung so gewählt, daß die gewünschte Zahl der Teststreifen realisierbar ist. Wenn die Vorrichtung mehrere Teststreifen umfaßt, sind diese vorzugsweise parallel zueinander angeordnet.

Entsprechende Teststreifen weisen üblicherweise eine Auftragezone auf, auf die die zu untersuchende flüssige Probe aufgetragen wird. Die Probe gelangt von dort in eine Reaktionszone, in der der nachzuweisende Analyt beispielsweise eine Farbreaktion auslöst. Das Auftreten einer Färbung in der Reaktionszone bedeutet dann das Vorliegen eines nachzuweisenden Analyten in der Probe. Alternativ kann aber auch, in Abhängigkeit von der Ausgestaltung des Teststreifens, keine Färbung oder eine geringe Färbung ein positives Testergebnis indizieren.

Um die Handhabung der erfindungsgemäßen Vorrichtung soweit wie möglich zu vereinfachen, und insbesondere zu verhindem, daß der Probennehmer das mit der Probe vollgesaugte Kissen weiter handhaben oder gar berühren muß, um die Probe mit dem Nachweissystem für den nachzuweisenden Analyten in Kontakt zu bringen, kann die erfindungsgemäße Vorrichtung eine Auspreßeinrichtung umfassen, die beim Bewegen der Gehäuseteile in ihre zweite Position, in der sie das saugfähige Kissen umschließen, dieses auspreßt.

Bei der Auspreßeinrichtung kann es sich beispielsweise um eine Erhebung in einem der beiden Gehäusteile handeln, die beim Bewegen der beiden Gehäuseteile in ihre zweite Position, in der sie das saugfähige Kissen umschließen, auf das saugfähige Kissen drückt und dieses dabei auspreßt. Vorteilhaft umfaßt das zweite Gehäuseteil ein Gegenstück für die Erhebung in dem ersten Gehäuseteil, damit der von der Erhebung ausgeübte Druck das saugfähige Kissen nicht nur von sich weg drückt sondern an das Gegenstück in dem zweiten Gehäuseteil andrückt und somit auspreßt. Dies ermöglicht, daß die in dem saugfähigen Kissen gesammelte Probe beim Verschließen der Gehäuseteile automatisch aus dem Kissen ausgepreßt und einer weiteren Analyse zur Verfügung gestellt wird. Eine besondere Handhabung oder Berührung durch den Probennehmer ist nicht erforderlich. Der Probennehmer kann die erfindungsgemäße Vorrichtung vielmehr einfach und sicher verschließen und gleichzeitig die gesammelte Probe für die weitere Analyse verfügbar machen, ohne mit der Probe in Kontakt zu kommen.

Um eine automatische Applikation der Probe auf den oder die Teststreifen zu ermöglichen, sollten diese so in einem der Gehäuseteile angeordnet sein, daß beim Bewegen der Gehäuseteile in ihre zweite Position, in der sie das saugfähige Kissen umschließen, die Auspreßeinrichtung die flüssige Probe aus dem saufähigen Kissen auf die Auftragezone der Teststreifen preßt. Hierdurch wird die Probe beim Verschließen des Gehäuses automatisch an der richtigen Stelle und in richtiger Menge auf die Auftragezone der Teststreifen aufgebracht, so daß reproduzierbare Testergebnisse gewährleistet sind.

Um den oder die Teststreifen bei geöffnetem Gehäuse, d.h. wenn sich die Gehäuseteile in ihrer ersten Position befinden, beispielsweise vor einem unbeabsichtigten Berühren oder Verunreinigen zu schützen, sind die Teststreifen vorzugsweise in einem der Gehäuseteile zwischen der Gehäusewand und einer Trennwand angeordnet. Diese Trennwand kann eine Einlaßöffnung aufweisen, durch die die flüssige Probe beim Auspressen aus dem saugfähigen Kissen während des Verschließens des Gehäuses zu den Teststreifen gelangen kann. Bei dieser Einlaßöffnung kann es sich beispielsweise um ein oder mehrere Löcher in der Trennwand handeln. Alternativ kann die Trennwand auch eine größere Öffnung aufweisen, die dann jedoch vorzugsweise ein Gitter oder einen porösen Körper enthält, gegen den das saugfähige Kissen beim Verschließen des Gehäuses gepreßt werden kann, um die Probe aus dem saugfähigen Kissen auszupressen. Die Löcher in der Trennwand, dem Gitter oder dem porösen Körper sollten so dimensioniert sein, daß die Probe leicht durch sie hindurchtreten kann. Bei einer gering viskosen Probe können die Löcher relativ klein sein. Bei einer viskoseren Probe wie beispielsweise Speichel sollten die Löcher entsprechend größer ausgestaltet sein.

In einer weiteren Ausführungsform umfaßt eines der Gehäuseteile der erfindungsgemäßen Vorrichtung eine Vorratskammer für eine Flüssigkeit. Diese Vorratskammer kann so angeordnet sein, daß sie beim Bewegen der Gehäuseteile in ihre zweite Position, d.h. beim Verschließen des Gehäuses auf der einen Seite des saugfähigen Kissens angeordnet ist und die Auftragezone der Teststreifen auf der anderen Seite des saugfähigen Kissens. Die Vorratskammer kann dadurch beispielsweise als Auspreßeinrichtung wirken und gleichzeitig die Probe aus dem Kissen auspressen und zusätzlich eine Flüssigkeit abgeben, die zum Auswaschen des Kissens beiträgt und die für den nachfolgenden Test zur Verfügung stehende Flüssigkeitsmenge erhöht. Außerdem kann es sich bei der Flüssigkeit in der Vorratskammer beispielsweise um eine Lösung wie eine Pufferlösung handeln, die den in der Probe vorhandenen Analyten stabilisiert oder in sonstiger Weise den nachfolgenden Test unterstützt.

Die Vorratskammer kann beispielsweise als Schwamm ausgestaltet sein, der beim Verschließen des Gehäuses gegen das saugfähige Kissen drückt und dabei die in dem Schwamm enthaltene Flüssigkeit freigibt. Vorteilhaft kann es sich bei der Vorratskammer beispielsweise auch um eine geschlossene Kammer handeln, die beispielsweise mit einem Blisterverschluß versehen ist. Hierdurch wird ein vorzeitiges Austreten der Flüssigkeit oder gar ein vollständiges Austrocknen verhindert. Der Blisterverschluß kann dann beispielsweise beim Schließen des Gehäuses automatisch durch eine geeignete Vorrichtung, wie z.B. eine Nadel oder eine Spitze, geöffnet werden, so daß die Flüssigkeit aus der Vorratskammer austreten kann.

Wenn die erfindungsgemäße Vorrichtung mit mindestens einem Teststreifen für den Nachweis mindestens eines Analyten in der flüssigen Probe ausgestattet ist, umfaßt sie vorzugsweise ein Sichtfenster, durch das der oder die Teststreifen beobachtet werden kann/können. Dies ermöglicht, daß das Testergebnis, das beispielsweise durch eine Verfärbung in der Reaktionszone der Teststreifen angezeigt wird, ohne ein erneutes Öffnen des Gehäuses abgelesen werden kann. Hierzu sollten die Teststreifen so nebeneinander angeordnet sein, daß sie gleichzeitig durch das Sichtfenster hindurch beobachtet werden können. Bei dem Sichtfenster kann es sich um eine Aussparung in einem der Gehäuseteile handeln. Dies hat jedoch den Nachteil, daß das Gehäuse nach dem Bewegen der beiden Gehäuseteile in ihre zweite Position nicht vollständig geschlossen ist, sondern beispielsweise Restflüssigkeit austreten kann. Daher ist das Sichtfenster vorzugsweise mit einem lichtdurchlässigen Material wie einer Glasscheibe oder eine Klarsichtfolie verschlossen. Dies hat außerdem den Vorteil, daß wenn der oder die Teststreifen mittels einer Ausleseoptik automatisch abgelesen werden soll(en), die reflektiven Eigenschaften der Teststreifen, die vielfach aus Nitrocellulose bestehen und daher zu einer diffusen Lichtstreuung führen, verbessert werden. Außerdem verhindert ein lichtdurchlässiges Material zwischen den Teststreifen und der Ausleseoptik ein Beschlagen der Ausleseoptik, selbst wenn ein relativ großes Probevolumen auf den Teststreifen aufgebracht wurde und diese daher feucht sind.

Das Sichtfenster kann entweder in dem Gehäuseteil vorgesehen sein, in dem sich auch der oder die Teststreifen befinden. Alternativ kann sich das Sichtfenster in dem Gehäuseteil befinden, das dem Gehäuseteil mit dem oder den Teststreifen gegenüberliegt. In diesem Fall muß beispielsweise durch zusätzliche Aussparungen in der Trennwand, unter der sich der oder die Teststreifen befinden, und dem Halter für das saugfähige Kissen dafür Sorge getragen werden, daß die Reaktionszone der Teststreifen durch das Sichtfenster beobachtet werden kann. Wenn mehrere Aussparungen vorgesehen sind, kann keine, mehrere oder alle dieser Aussparungen mit einem lichtdurchlässigen Material verschlossen sein. Femer kann sich zumindest ein Teststreifen in dem Gehäuseteil befinden, der das Sichtfenster umfaßt, und mindestens ein weiterer Teststreifen kann sich in dem anderen Gehäuseteil befinden, das gegebenenfalls mit einem zweiten Sichtfenster versehen sein kann.

Die Größe des Sichtfensters ist von keiner besonderen Bedeutung, sie sollte jedoch so sein, daß zumindest die Reaktionszone des oder der Teststreifen(s) beobachtet werden kann. Alternativ ist es auch möglich, beispielsweise eines oder beide der Gehäuseteile oder die gesamte erfindungsgemäße Vorrichtung aus einem durchsichtigen Material herzustellen. In diesem Fall kann für die Beobachtung des oder der Teststreifen(s) auf ein Sichtfenster verzichtet werden.

Das Gehäuse und die Halterung für das saugfähige Kissen können im Prinzip aus beliebigen Materialien hergestellt werden. Vorzugsweise bestehen sie aus Kunststoff, wie beispielsweise Polypropylen oder Polyethylen, der beispielsweise durch Spritzgießen in die gewünschte Form gebracht wird.

In einer weiteren bevorzugten Ausführungsform ist die erfindungsgemäße Vorrichtung so ausgestaltet, daß zunächst ein unteres Gehäuseteil unter das saugfähige Kissen geklappt wird und erst anschließend das obere Gehäuseteil zum Verschließen des Gehäuses über das saugfähige Kissen geklappt wird. Dies erleichtert weiter die Handhabung der erfindungsgemäßen Vorrichtung und trägt dazu bei, daß der Probennehmer mit der in dem saugfähigen Kissen aufgesaugten Probe nicht in Kontakt kommt. Dies erlaubt ein hygienisch einwandfreies Handhaben der erfindungsgemäßen Vorrichtung und erhöht die Akzeptanz bei nichttrainierten Anwendern, wie beispielsweise Polizisten während Routinekontrollen, beispielsweise auf Drogenmißbrauch.

Die erfindungsgemäße Vorrichtung eignet sich außerdem als Einwegtest, bei dem zunächst eine Probe genommen und dann direkt in der Vorrichtung eingeschlossen wird, ohne daß überschüssige Lösung austreten kann oder nachträgliche Manipulationen des Tests möglich sind. Dies ermöglicht eine sichere Aufbewahrung der Testprobe bei gleichzeitiger Möglichkeit der Ablesung des Testergebnisses. Um mögliche Manipulationen der Testprobe zu verhindern, kann die erfindungsgemäße Vorrichtung zusätzlich eine Anzeigeeinrichtung enthalten, die ein Öffnen des Gehäuses, nachdem die Gehäuseteile in ihre zweite Position, in der sie das saugfähige Kissen einschließen, bewegt worden sind, anzeigt. Bei einer solchen Anzeigevorrichtung kann es sich beispielsweise um einen Schnappverschluß handeln, der nach seinem Einrasten nur durch ein Zerbrechen wieder geöffnet werden kann.

Die erfindungsgemäße Vorrichtung wird nun anhand der beiliegenden Figuren, die ein bevorzugtes Ausführungsbeispiel zeigen, ohne die Erfindung jedoch darauf zu beschränken, näher erläutert. In den anliegenden Figuren zeigen:
**Figur 1** eine Explosionszeichnung der erfindungsgemäßen Vorrichtung mit geöffnetem Gehäuse in perspektivischer Ansicht von unten,
**Figur 2** eine perspektivische Ansicht der geöffneten erfindungsgemäßen Vorrichtung von oben,
**Figur 3** eine perspektivische Ansicht der geschlossenen erfindungsgemäßen Vorrichtung von oben,
**Figur 4** eine Seitenansicht der geöffneten erfindungsgemäßen Vorrichtung,
**Figur 5** eine Seitenansicht der geschlossenen erfindungsgemäßen Vorrichtung,
**Figur 6** die geschlossene erfindungsgemäßen Vorrichtung von oben und
**Figur 7** die geschlossene erfindungsgemäße Vorrichtung von oben, jedoch ohne das obere Gehäuseteil.

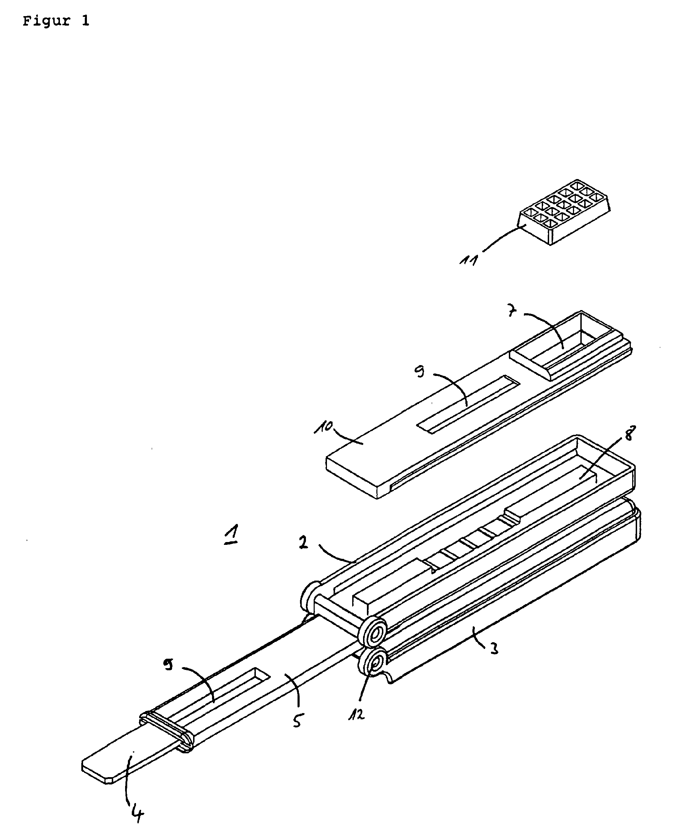

In Figur **1** ist die erfindungsgemäße Vorrichtung **1** in einer bevorzugten Ausführungsform als Explosionszeichnung perspektivisch wiedergegeben. Das saugfähige Kissen **4** ist an einem Halter **5** befestigt, der mit dem Gehäuseunterteil **2** und dem Gehäuseoberteil **3** verbunden ist. Die beiden Gehäuseteile sind über die Gelenke **12** miteinander verbunden und können von der dargestellten ersten Position, in der das saugfähige Kissen **4** eine flüssige Probe aufnehmen kann, über die Gelenke **12** in eine zweite Position geklappt werden, in der sie das saugfähige Kissen **4** umschließen. Das Gehäuseteil **2** umfaßt einen Teststreifen **8,** der sich zwischen der Wand des Gehäuseteils **2** und der Trennwand **10** befindet. Die Trennwand **10** umfaßt eine Einlaßöffnung **7,** die mit einem Gitter **11** versehen ist. Beim Schließen des Gehäuses wird das saugfähige Kissen **4** mittels einer in Figur 1 nicht dargestellten Auspreßvorrichtung **6** in dem Gehäuseoberteil **3** gegen das Gitter **11** gepreßt, so daß eine zuvor in das Kissen **4** aufgenommene flüssige Probe durch das Gitter **11** zum dem Teststreifen **8** gelangt. Die Trennwand 10, der Halter **5** und (in Figur 1 nicht dargestellt) das Gehäuseoberteil **3** weisen Sichtfenster **9** auf, durch die der Teststreifen 8 beobachtet werden kann.

Bei der Anwendung der in Figur 1 dargestellten erfindungsgemäßen Vorrichtung beispielsweise zum Sammeln einer Speichelprobe wird das saugfähige Kissen **4** an dem Halter **5** von der Testperson so lange in den Mund genommen, bis sich das saugfähige Kissen **4** mit dem Speichel der Testperson vollgesogen hat. Hierfür reicht im allgemeinen eine Zeitdauer von ca. einer halben Minute aus, wobei diese Zeitdauer jedoch von der Saugfähigkeit und der Größe des Kissens **4** sowie dem Vorhandensein von Speichel und dem Speichelfluß abhängt. Vorzugsweise sollte das saugfähige Kissen **4** ca. 200-400 µl, insbesondere etwa 300 µl Speichel aufnehmen. Hierfür würde sich beispielsweise ein ca. 1 cm langes, 6 mm breites und 1,5-2 mm dickes saugfähiges Kissen **4** aus gepreßter Cellulose eignen. Nachdem sich das saugfähige Kissen **4** mit dem Speichel der Testperson vollgesogen hat, kann die erfindungsgemäße Vorrichtung von dem Probennehmer an den Gehäuseteilen **2** und **3** gehalten werden, ohne daß der Probennehmer mit dem speichelgetränkten saugfähigen Kissen **4** in Kontakt kommt. Vorzugsweise wird dann zunächst eines der Gehäuseteile umgeklappt, so daß es unter dem saugfähigen Kissen **4** und dem Halter **5** zu liegen kommt. Anschließend wird das andere Gehäuseteil ebenfalls umgeklappt, so daß das saugfähige Kissen **4** und der Halter **5** zwischen den beiden Gehäuseteilen **2** und **3** eingeschlossen werden. Alternativ können auch beide Gehäuseteile **2** und **3** gleichzeitig umgeklappt werden.

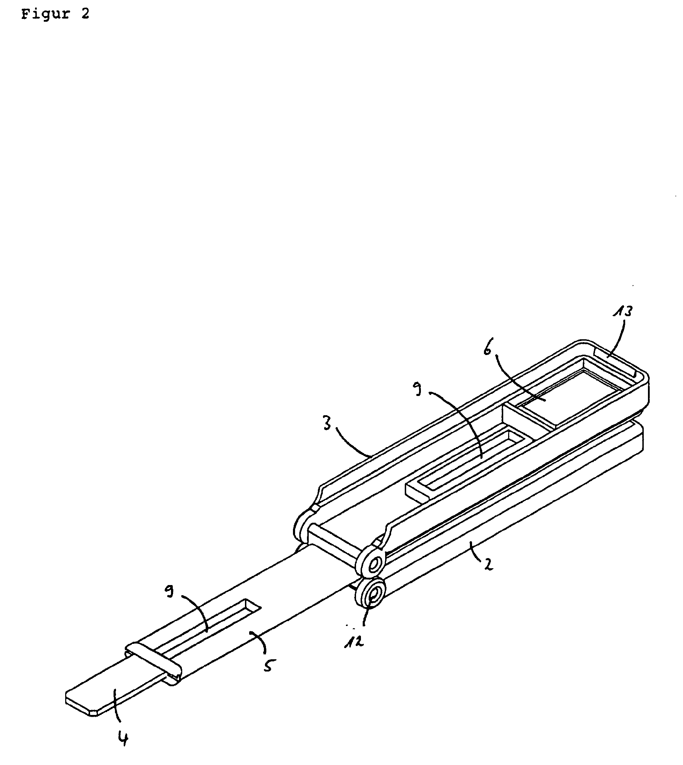

Beim Verschließen der beiden Gehäuseteile **2** und **3** wird das speichelgetränkte saugfähige Kissen **4** mittels einer in Figur 2 dargestellten Auspreßvorrichtung **6** am Gehäuseobererteil **3** gegen das Gitter **11** in der Einlaßöffnung **7** der Trennwand **10** gedrückt, wodurch der Speichel aus dem saugfähigen Kissen **4** durch das Gitter **11** auf den Teststreifen **8** übertragen wird. Von der Auftragezone des Teststreifens **8** wandert der Speichel in die Reaktionszone des Teststreifens, wo er, wenn ein entsprechender Analyt, wie beispielsweise ein Droge, in ihm vorliegt, eine Verfärbung auslöst. Diese Verfärbung des Teststreifens kann durch die Sichtfenster **9** beobachtet werden, ohne daß das Gehäuse geöffnet werden müßte. Die Auspreßvorrichtung **6** kann zusätzlich oder alternativ eine Vorratskammer für eine Flüssigkeit sein.

In Figur 2 ist zusätzlich ein Schnappverschluß **13** an dem Gehäuseoberteil **3** gezeigt, der in eine entsprechende Aussparung des Gehäuseunterteils **2** beim Verschließen des Gehäuses eingreift. Hierdurch wird ein erneutes Öffnen des Gehäuses verhindert.

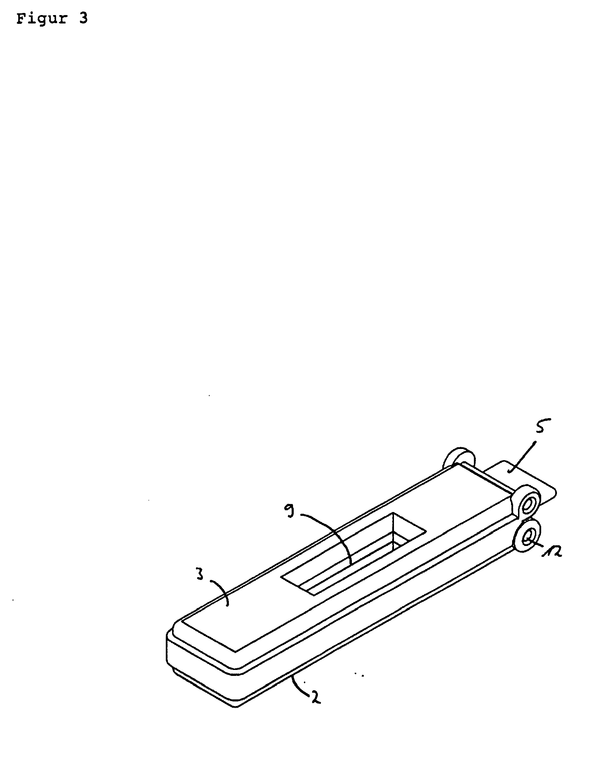

Figur 3 zeigt eine perspektivische Darstellung der in den Figuren 1 und 2 gezeigten erfindungsgemäßen Vorrichtung in geschlossenem Zustand. Man erkennt die Gehäuseteile **2** und **3**, die über die Gelenke **12** miteinander verbunden sind und die Sichtfenster **9,** durch die der hier nicht sichtbare Teststreifen beobachtet werden kann.

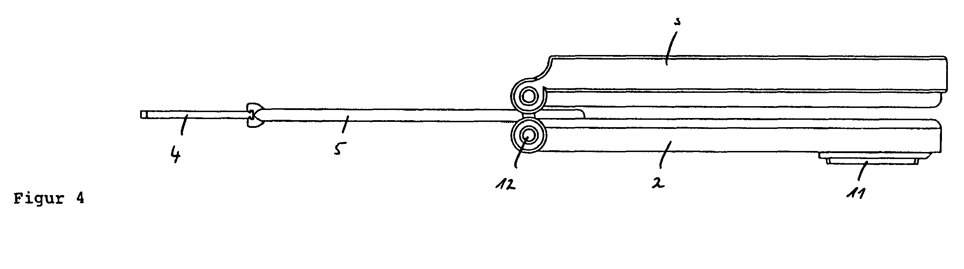

Figur 4 zeigt die erfindungsgemäße Vorrichtung mit geöffnetem Gehäuse von der Seite. Aus dem Gehäuseunterteil **2** ragt das Gitter **11** hervor, gegen das das saugfähige Kissen **4** beim Schließen der beiden Gehäuseteile durch das Gehäuseteil **3** gepreßt wird.

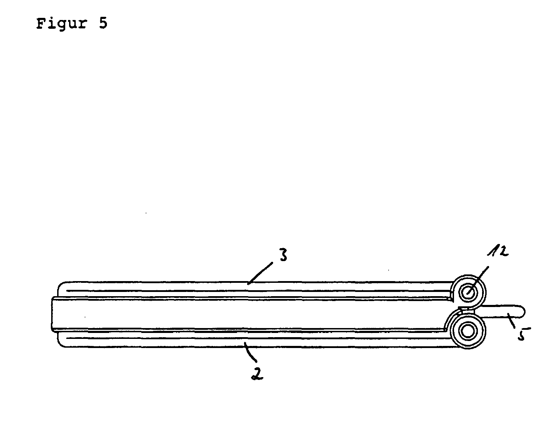

Figur 5 zeigt dieselbe Vorrichtung wie Figur 4 in geschlossenem Zustand.

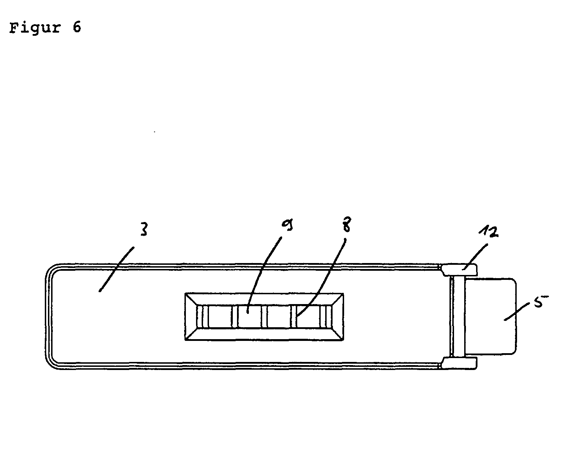

Figur 6 zeigt die erfindungsgemäße Vorrichtung von oben. Man erkennt das Gehäuseoberteil **3** mit dem Sichtfenster **9** durch das der Teststreifen **8** sichtbar ist.

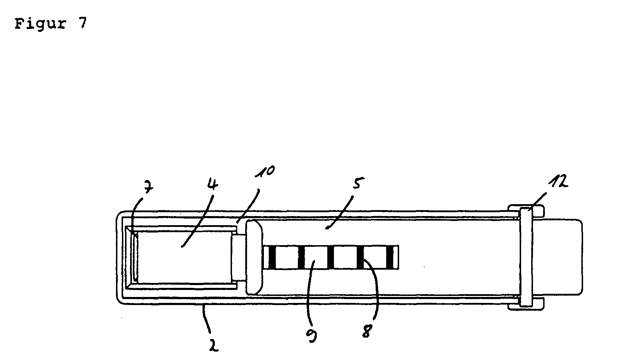

Figur 7 zeigt dieselbe Vorrichtung wie Figur 6 nur ohne Gehäuseoberteil **3.** Man erkennt das auf der Trennwand **10** des Gehäuseunterteils **2** aufliegende saugfähige Kissen **4** mit dem Halter **5.** Das saugfähige Kissen **4** befindet sich über der Einlaßöffnung **7.** In dem Halter **5** befindet sich ein Sichtfenster **9** durch das der Teststreifen **8** beobachtet werden kann.

## Patentansprüche

1. Vorrichtung zum Sammeln von flüssigen Proben, wobei die Vorrichtung ein saugfähiges Kissen (4) und zwei Gehäuseteile (2, 3) umfaßt, die mit dem saugfähigen Kissen (4) beweglich verbunden sind, wobei die Gehäuseteile (2, 3) derart angeordnet sind, daß in einer ersten Position der Gehäuseteile (2, 3) das saugfähige Kissen (4) die flüssige Probe aufnehmen kann und in einer zweiten Position die Gehäuseteile (2, 3) das saugfähige Kissen (4) umschließen, **dadurch gekennzeichnet, daß** das saugfähige Kissen (4) an einem Halter (5) befestigt ist, der über mindestens ein Gelenk (12) mit den beiden Gehäuseteilen (2, 3) verbunden ist.

2. Vorrichtung nach Anspruch 1, wobei die Vorrichtung zum Sammeln von Körperflüssigkeiten angepaßt ist.

3. Vorrichtung nach Anspruch 2, wobei die Vorrichtung zum Sammeln von Speichel aus der Mundhöhle angepaßt ist.

4. Vorrichtung nach einem der vorhergehenden Ansprüche, wobei das saugfähige Kissen (4) gepreßte Cellulose umfaßt.

5. Vorrichtung nach einem der vorhergehenden Ansprüche, wobei das saugfähige Kissen (4) ein den Speichelfluß anregendes Mittel umfaßt.

6. Vorrichtung nach einem der vorhergehenden Ansprüche, umfassend mindestens einen Teststreifen (8) für den Nachweis mindestens eines Analyten in der flüssigen Probe.

7. Vorrichtung nach einem der vorhergehenden Ansprüche, wobei die Gehäuseteile (2, 3) eine Auspreßeinrichtung (6) umfassen, die beim Bewegen der Gehäuseteile in ihre zweite Position, in der sie das saugfähige Kissen (4) umschließen, dieses auspreßt.

8. Vorrichtung nach Anspruch 6 und 7, wobei der oder die Teststreifen (8) eine Auftragezone für die flüssige Probe aufweisen und der oder die Teststreifen (8) so in einem der Gehäuseteile (2, 3) angeordnet sind, das beim Bewegen der Gehäuseteile (2, 3) in ihre zweite Position, in der sie das saugfähige Kissen (4) umschließen, die Auspreßeinrichtung (6) die flüssige Probe aus dem saugfähigen Kissen (4) auf die Auftragezone des oder der Teststreifen(s) (8) preßt.

9. Vorrichtung nach einem der Ansprüche 6-8, wobei der oder die Teststreifen (8) in einem der Gehäuseteile (2, 3) zwischen der Gehäusewand und einer Trennwand (10) angeordnet sind.

10. Vorrichtung nach Anspruch 9, wobei die Trennwand (10) eine Einlaßöffnung (7) aufweist, durch die flüssige Probe zu dem oder den Teststreifen (8) gelangen kann.

11. Vorrichtung nach einem der vorhergehenden Ansprüche, wobei eines der Gehäuseteile (2, 3) eine Vorratskammer für eine Flüssigkeit umfaßt.

12. Vorrichtung nach einem der Ansprüche 6-11, wobei die Vorrichtung mindestens ein Sichtfenster (9) umfaßt, durch das der oder die Teststreifen (8) beobachtet werden können.

13. Vorrichtung nach Anspruch 12, wobei das Sichtfenster (9) mit einem lichtdurchlässigen Material verschlossen ist.

14. Vorrichtung nach einem der vorhergehenden Ansprüche, umfassend eine Anzeigeeinrichtung, die ein Öffnen des Gehäuses nachdem die Gehäuseteile (2, 3) in ihre zweite Position, in der sie das saugfähige Kissen (4) umschließen, bewegt worden sind, anzeigt.

15. Verwendung einer Vorrichtung nach einem der vorhergehenden Ansprüche zum Sammeln einer Speichelprobe.

16. Verwendung einer Vorrichtung nach einem der Ansprüche 6-14 zum Sammeln einer Speichelprobe und Nachweisen eines Analyten in dieser Speichelprobe.

## Claims

1. A device for collecting samples of fluids, said device comprising an absorbent pad (4) and two housing parts (2, 3) which are connected to the absorbent pad (4) in a movable manner, said housing parts (2, 3) being arranged in such a way that, in a first position of the housing parts (2, 3), the absorbent pad (4) can take up the sample of fluid, and, in a second position, the housing parts (2, 3) surround the absorbent pad (4), **characterized in that** the absorbent pad (4) is secured on a holder (5) which is connected to the two housing parts (2, 3) via at least one hinge (12).

2. The device as claimed in claim 1, which device is adapted for collecting body fluids.

3. The device as claimed in claim 2, which device is adapted for collecting saliva from the oral cavity.

4. The device as claimed in one of the preceding claims, in which the absorbent pad (4) comprises pressed cellulose.

5. The device as claimed in one of the preceding claims, in which the absorbent pad (4) comprises a means stimulating the flow of saliva.

6. The device as claimed in one of the preceding claims, comprising at least one test strip (8) for detection of at least one analyte in the sample of fluid.

7. The device as claimed in one of the preceding claims, in which the housing parts (2, 3) comprise a squeezing arrangement (6) which, when the housing parts are moved into their second position in which they surround the absorbent pad (4), squeezes the fluid out of the latter.

8. The device as claimed in claims 6 and 7, in which the test strip or test strips (8) has/have an application zone for the sample of fluid, and the test strip or test strips (8) is/are arranged in one of the housing parts (2, 3) in such a way that, when the housing parts (2, 3) are moved into their second position in which they surround the absorbent pad (4), the squeezing arrangement (6) squeezes the sample of fluid out of the absorbent pad (4) onto the application zone of the test strip(s) (8).

9. The device as claimed in one of claims 6-8, in which the test strip or test strips (8) is/are arranged in one of the housing parts (2, 3) between the housing wall and a dividing wall (10).

10. The device as claimed in claim 9, in which the dividing wall (10) has an inlet opening (7) through which the sample of fluid can pass to the test strip or test strips (8).

11. The device as claimed in one of the preceding claims, in which one of the housing parts (2, 3) comprises a storage chamber for a liquid.

12. The device as claimed in one of claims 6-11, in which the device comprises at least one viewing window (9) through which the test strip or test strips (8) can be observed.

13. The device as claimed in claim 12, in which the viewing window (9) is closed with a transparent material.

14. The device as claimed in one of the preceding claims, comprising a display means which indicates opening of the housing after the housing parts (2, 3) have been moved to their second position in which they surround the absorbent pad (4).

15. Use of the device as claimed in one of the preceding claims, for collecting a sample of saliva.

16. Use of the device as claimed in one of claims 6-14, for collecting a sample of saliva and for detecting an analyte in this sample of saliva.

## Revendications

1. Dispositif pour collecter des échantillons liquides, le dispositif comprenant un coussin hydrophile (4) et deux pièces de boîtier (2, 3) qui sont reliées de manière mobile au coussin hydrophile (4), les pièces de boîtier (2, 3) étant disposées de telle sorte que, dans une première position des pièces de boîtier (2, 3), le coussin hydrophile (4) peut absorber l'échantillon liquide et que, dans une seconde position, les pièces de boîtier (2, 3) entourent le coussin hydrophile (4), **caractérisé en ce que** le coussin hydrophile (4) est fixé sur un support (5) qui est relié par l'intermédiaire d'au moins une articulation (12) aux deux pièces de boîtier (2, 3).

2. Dispositif selon la revendication 1, le dispositif étant adapté pour collecter des liquides biologiques.

3. Dispositif selon la revendication 2, le dispositif étant adapté pour collecter de la salive à partir de la cavité buccale.

4. Dispositif selon l'une des revendications précédentes, le coussin hydrophile (4) comprenant de la cellulose pressée.

5. Dispositif selon l'une des revendications précédentes, le coussin hydrophile (4) comprenant un agent stimulant le flux de salive.

6. Dispositif selon l'une des revendications précédentes, comprenant au moins une bandelette réactive (8) pour déceler au moins un analyte dans l'échantillon liquide.

7. Dispositif selon l'une des revendications précédentes, les pièces de boîtier (2, 3) comprenant un dispositif de pression (6) qui, lors du déplacement des pièces de boîtier dans leur seconde position, dans laquelle elles entourent le coussin hydrophile (4), presse celui-ci.

8. Dispositif selon les revendications 6 et 7, la ou les bandelette(s) réactive(s) (8) présentant une zone d'application pour l'échantillon liquide et la ou les bandelette(s) réactive(s) (8) étant disposée(s) dans une des pièces de boîtier (2, 3) de sorte que lors du déplacement des pièces de boîtier (2, 3) dans leur seconde position, dans laquelle elles entourent le coussin hydrophile (4), le dispositif de pression (6) presse l'échantillon liquide en dehors du coussin hydrophile (4) sur la zone d'application de la ou des bandelette(s) réactive(s) (8).

9. Dispositif selon l'une des revendications 6 à 8, la ou les bandelette(s) réactive(s) (8) étant disposée(s) dans l'une des pièces de boîtier (2, 3) entre la paroi du boîtier et une paroi de séparation (10).

10. Dispositif selon la revendication 9, la paroi de séparation (10) comprenant un orifice d'admission (7) à travers lequel l'échantillon liquide peut parvenir jusqu'à la ou les bandelette(s) réactive(s) (8).

11. Dispositif selon l'une des revendications précédentes, une des pièces de boîtier (2, 3) comprenant une chambre de réserve pour un liquide.

12. Dispositif selon l'une des revendications 6 à 11, le dispositif comprenant au moins une fenêtre (9) à travers laquelle la ou les bandelette(s) réactive(s) (8) peut/peuvent être observée(s).

13. Dispositif selon la revendication 12, la fenêtre (9) étant obturé à l'aide d'un matériau transparent.

14. Dispositif selon l'une des revendications précédentes, comprenant un témoin qui indique une ouverture du boîtier après le déplacement des pièces de boîtier (2, 3) dans leur seconde position, dans laquelle elles entourent le coussin hydrophile (4).

15. Utilisation d'un dispositif selon l'une des revendications précédentes pour collecter un échantillon de salive.

16. Utilisation d'un dispositif selon l'une des revendications 6 à 14 pour collecter un échantillon de salive et déceler un analyte dans cet échantillon de salive.
